# EUROPEAN PATENT APPLICATION

(11) **EP 4 682 261 A1**
(43) Date of publication of application: **21.01.2026**
(21) Application number: 24774791.8
(22) Date of filing: 13.03.2024
(51) Int. Cl.: C12P 13/02

(54) **METHOD FOR PRODUCING AMIDE COMPOUND**

(30) Priority: 17.03.2023 JP 2023043453
(71) Applicant: Mitsubishi Chemical Corporation, Tokyo 100-8251 (JP)
(72) Inventor: KANO, Makoto, Tokyo 100-8251 (JP); YAMAGUCHI, Takafumi, Tokyo 100-8251 (JP)
(74) Representative: Hoffmann Eitle
(86) International application number: PCT/JP2024/009781
(87) International publication number: WO 2024/195653

(57) **Abstract**

A method for producing an amide compound from a nitrile compound using a biocatalyst by producing an amide compound aqueous solution having low foamability. In the method for producing an amide compound by performing a hydration reaction on a nitrile compound in the presence of a biocatalyst having nitrile hydratase activity, the pH of a reaction solution is increased in the middle of the hydration reaction. In one example, the hydration reaction is performed in a state in which the pH of the reaction solution in the latter half of the reaction, at which a contained amount of amide compound is 40% by mass or more with respect to the total mass of the reaction solution, is set to be higher than the pH of the reaction solution in the former half of the reaction, at which the contained amount of amide compound is less than 40% by mass with respect to the total mass of the reaction solution.

## Description

### TECHNICAL FIELD

The present invention relates to a method for producing an amide compound from a nitrile compound using a biocatalyst having nitrile hydratase activity.

Priority is claimed on Japanese Patent Application No. 2023-043453, filed March 17, 2023, the content of which is incorporated herein by reference.

### BACKGROUND ART

In recent years, a method for producing a compound using a biocatalyst has been used for producing many compounds because of advantages such as mild reaction conditions, simplification of a reaction process, and high purity of a reaction product due to a small amount of by-products.

In the production of an amide compound, since a nitrile hydratase as an enzyme which converts a nitrile compound into an amide compound has been found, the use of a biocatalyst has been actively studied (Patent Documents 1 to 6, and the like). In addition, the process has also been actively studied, and many proposals have been made regarding batchwise reaction, semi-batchwise reaction, multi-stage continuous reaction, pipe reactor, and the like.

For example, Patent Document 5 discloses the difference between a cooling water temperature and a reaction temperature for efficiently removing reaction heat, and discloses a method for producing an amide compound at low cost by reducing energy cost.

In addition, Patent Document 6 discloses a stirring power and a fluid number that can efficiently mix a reaction solution while preventing volatilization of acrylonitrile during the reaction, and discloses a method for producing an amide compound from a nitrile compound at low cost.

### Citation List

### Patent Documents

Patent Document 1: Japanese Unexamined Patent Application, First Publication No. H11-123098
Patent Document 2: Japanese Unexamined Patent Application, First Publication No. H7-265091
Patent Document 3: Japanese Examined Patent Application, Second Publication No. S56-38118
Patent Document 4: Japanese Unexamined Patent Application, First Publication No. H11-89575
Patent Document 5: PCT International Publication No. WO2010/038832
Patent Document 6: PCT International Publication No. WO2009/113654

### SUMMARY OF INVENTION

### Technical Problem

However, in order to industrially use the biocatalyst, it is desired not only to produce an amide compound at a low cost but also to produce an amide compound which satisfies a certain desired quality. Since an amide compound aqueous solution produced by a biocatalytic method contains a protein derived from a biocatalyst as an impurity, there is a problem in that the obtained aqueous amide compound aqueous solution is likely to foam. Therefore, it is difficult to handle the amide compound aqueous solution when transferring, transporting, and storing the amide compound aqueous solution. Furthermore, when the amide compound is polymerized to obtain an amide compound-based polymer, there is a problem in that the amide compound aqueous solution overflows from a polymerization kettle due to foaming, and thus the yield of the amide compound-based polymer is reduced.

In the methods of Patent Documents 1 to 6, foaming of the amide compound aqueous solution due to the protein as an impurity cannot be sufficiently suppressed.

A main object of the present invention is to provide a method for producing an amide compound aqueous solution having low foamability.

### Solution to Problem

The present inventors have conducted intensive studies in view of the problems of the related art, and as a result, have found that foaming of the obtained amide compound aqueous solution can be suppressed by increasing the pH of a reaction solution in the middle of the hydration reaction, thus achieving the present invention. That is, the present invention relates to the following [1] to [8].

[1] A method for producing an amide compound, comprising:
   performing a hydration reaction on a nitrile compound in the presence of a biocatalyst having nitrile hydratase activity,
   wherein the pH of a reaction solution is increased in a middle of the hydration reaction.
[2] The method for producing an amide compound according to [1],
   wherein the pH of the reaction solution is increased in the middle of the reaction to be higher than the pH at the start of the reaction.
[3] The method for producing an amide compound according to [1] or [2],
   wherein the hydration reaction is performed in a state in which the pH of the reaction solution in the latter half of the reaction, at which a contained amount of amide compound is 40% by mass or more with respect to a total mass of the reaction solution, is set to be higher than the pH of the reaction solution in at least part of the former half of the reaction, at which the contained amount of amide compound is less than 40% by mass with respect to the total mass of the reaction solution.
[4] The method for producing an amide compound according to any one of [1] to [3],
   wherein the pH of the reaction solution is increased by 0.3 to 1.5.
[5] The method for producing an amide compound according to any one of [1] to [4],
   wherein the pH of the reaction solution at the start of the reaction is 6.6 to 7.5.
[6] The method for producing an amide compound according to any one of [3] to [5],
   wherein the hydration reaction is performed in a state in which the pH of the reaction solution in the former half of the reaction is set to 6.6 to 7.5 and the pH of the reaction solution in the latter half is set to 7.5 or more and less than 8.5.
[7] The method for producing an amide compound according to any one of [1] to [6],
   wherein the nitrile compound is acrylonitrile or methacrylonitrile.
[8] The method for producing an amide compound according to any one of [1] to [6], wherein the amide compound is acrylamide or methacrylamide.

### Advantageous Effects of Invention

With the method according to the present invention, in a method for producing an amide compound from a nitrile compound using a biocatalyst, by increasing the pH of the reaction solution in the middle of the hydration reaction, it is possible to suppress foaming of the obtained reaction solution, that is, an amide compound aqueous solution, and thus handling of the reaction solution is facilitated.

### DESCRIPTION OF EMBODIMENTS

In the present specification, a numerical range represented by "to" means a numerical range including numerical values before and after "to" as a lower limit value and an upper limit value.

In the numerical ranges of contained amounts, various physical property values, and property values described in the present specification, the lower limit value and the upper limit value thereof can be randomly combined to form a new numerical range.

The method for producing an amide compound according to the embodiment is a method for producing an amide compound by performing a hydration reaction of a nitrile compound in the presence of a biocatalyst having nitrile hydratase activity. In one example of the embodiment of the present aspect, the pH of a reaction solution is increased in the middle of the hydration reaction.

Hereinafter, an example of the embodiment of the present aspect will be described.

### (1) Biocatalyst having nitrile hydratase activity

In the present embodiment, nitrile hydratase refers to an enzyme having an ability to hydrolyze a nitrile compound to generate a corresponding amide compound. The biocatalyst having nitrile hydratase activity may be a nitrile hydratase protein itself, but may be an animal cell, a plant cell, a cell organelle, or a microbial cell body, which contains nitrile hydratase, and a treated product thereof.

Examples of the above-described treated product include animal cells, plant cells, cell organelles, a crushed product obtained by crushing a microbial cell body, or an enzyme (a crude enzyme or a purified enzyme) extracted from a microbial cell body; and a carrier on which animal cells, plant cells, cell organelles, a microbial cell body, or an enzyme itself is immobilized.

In addition, the above-described treated product also includes an animal cell, a plant cell, a cell organelle, or a microbial cell body which has lost proliferation ability due to a drug treatment. The microbial cell body which has lost proliferation ability due to a drug treatment is also referred to as "dead cell body".

Examples of the immobilization method include an inclusion method, a crosslinking method, and a carrier bonding method. The inclusion method is a method of coating with a polymer. The crosslinking method is a method of crosslinking an enzyme with a reagent having two or more functional groups, that is, a polyfunctional crosslinking agent. The carrier bonding method is a method of bonding an enzyme to an insoluble carrier.

Examples of the carrier used for the immobilization include glass beads, silica gel, polyurethane, polyacrylamide, polyvinyl alcohol, carrageenan, alginic acid, agar, and gelatin.

Representative examples of such microorganisms include microorganisms belonging to the genera Rhodococcus, Gordona, Pseudomonas, Pseudonocardia, Geobacillus, Bacillus, Bacteridium, Micrococcus, Brevibacterium, Corynebacterium, Nocardia, Microbacterium, Fusarium, Agrobacterium, Acinetobacter, Xanthobacter, Streptomyces, Rhizobium, Klebsiella, Enterobacter, Erwinia, Pantoea, Candida, Aeromonas, Citrobacter, and Achromobacter, which have the nitrile hydratase activity.

More specific examples thereof include Nocardia sp. N-775 described in Japanese Examined Patent Application, Second Publication No. S56-17918; Rhodococcus rhodochrous J-1 described in Japanese Examined Patent Application, Second Publication No. H06-55148; Rhodococcus rhodochrous NCIMB41164 strain described in PCT International Publication No. WO2005/054456; Klebsiella sp. MCI2609 described in Japanese Unexamined Patent Application, First Publication No. H05-30982; Aeromonas sp. MCI2614 described in Japanese Unexamined Patent Application, First Publication No. H05-30983; Citrobacter freundii MCI2615 described in Japanese Unexamined Patent Application, First Publication No. H05-30984; Agrobacterium rhizogenes IAM13570 and Agrobacterium faciens described in Japanese Unexamined Patent Application, First Publication No. H05-103681; Xanthobacter flavus JCM1204 and Erwinia nigrifluens MAFF 03-01435 described in Japanese Unexamined Patent Application, First Publication No. H05-161495; Enterobacter sp. MCI2707 described in Japanese Unexamined Patent Application, First Publication No. H05-236975; Streptomyces sp. MCI2691 described in Japanese Unexamined Patent Application, First Publication No. H05-236976; Rhizobium sp. MCI2610, Rhizobium sp. MCI2643, Rhizobium loti IAM13588, Rhizobium leguminosarum IAM12609, and Rhizobium meriotii IAM12611 described in Japanese Unexamined Patent Application, First Publication No. H05-236977; Candida guilliermondii NH-2, Pantoea agglomerans NH-3, and Klebsiella pneumoniae subsp. pneumoniae NH-26T2 described in Japanese Unexamined Patent Application, First Publication No. H05-15384; Agrobacterium radiobacter SC-C15-1 described in Japanese Unexamined Patent Application, First Publication No. H06-14786; Bacillus smithii SC-J05-1 described in Japanese Unexamined Patent Application, First Publication No. H07-25494; Pseudonocardia thermophila ATCC19285 described in Japanese Unexamined Patent Application, First Publication No. H08-56684; and Pseudonocardia thermophila JCM3095 described in Japanese Unexamined Patent Application, First Publication No. H09-275978.

The Rhodococcus rhodochrous J-1 strain described in Japanese Examined Patent Application, Second Publication No. H06-55148 is deposited on September 18, 1987 under the accession number "FERM BP-1478" at the Patent Organism Depositary, National Institute of Technology and Evaluation (Chuo 6, 1-1-1 Higashi, Tsukuba-shi, Ibaraki-ken (hereinafter the same in this specification)).

The Rhodococcus rhodochrous NCIMB41164 strain described in PCT International Publication No. WO2005/054456 is deposited on March 5, 2003 under the accession number "NCIMB41164" at the National Collection of Industrial, Food and Marine Bacteria, Ltd. (NCIMB) (NCIMB Ltd Ferguson Building Craibstone Estate Buksburn Aberdeen AB21 9YA).

The Pseudonocardia thermophila JCM3095 described in Japanese Unexamined Patent Application, First Publication No. H09-275978 is deposited on February 7, 1996 under the accession number "FERM BP-5785" at the National Institute of Technology and Evaluation, Patent Organism Depositary (1-1-1 Central 6, Higashi, Tsukuba, Ibaraki Prefecture).

In the present embodiment, one kind of microorganism having a desired characteristic, selected from the above-described microorganisms, can be used alone, or two or more kinds thereof can be used in combination.

A gene encoding the nitrile hydratase can be introduced into and expressed in a microbial cell by a general molecular biological method. For these molecular biological methods, Sambrook, Fritsch, and Maniatis, "Molecular Cloning: A Laboratory Manual" 2nd Edition (1989), Cold Spring Harbor Laboratory Press can be referred to. That is, in the present embodiment, an enzyme obtained by expressing a nucleic acid encoding a natural nitrile hydratase (wild type) or a mutant thereof (improved type) in the microbial cell can also be used.

In the present embodiment, one kind selected from the above-described enzymes can be used alone, or two or more kinds thereof can be used in combination.

An amino acid sequence of the wild-type nitrile hydratase is disclosed in the NCBI database such as GenBank (http://www.ncbi.nlm.nih.gov/).

For example, Accession No. of an α-subunit derived from Rhodococcus rhodochrous J1 (FERM BP-1478) is "P21219", and Accession No. of a β-subunit is "P21220". In addition, Accession No. of an α-subunit derived from Rhodococcus rhodochrous M8 (SU1731814) is "ATT79340", and Accession No. of a β-subunit is "AAT79339". Furthermore, Accession No. of an α-subunit derived from Pseudomonas thermophila JCM3095 is "1IREA", and Accession No. of a β-subunit is "1IREB".

Examples of a transformant into which the wild-type nitrile hydratase gene has been introduced include Escherichia coli MT10770 (FERM P-14756) (Japanese Unexamined Patent Application, First Publication No. H8-266277) transformed with a nitrile hydratase of the genus Achromobacter; Escherichia coli MT10822 (FERM BP-5785) (Japanese Unexamined Patent Application, First Publication No. H9-275978) transformed with a nitrile hydratase of the genus Pseudonocardia; and a microorganism transformed with a nitrile hydratase of the Rhodococcus rhodochrous species (Japanese Unexamined Patent Application, First Publication No. H4-211379), but the transformant is not limited thereto.

The improved (mutant)-type nitrile hydratase in which the wild-type nitrile hydratase is subjected to amino acid substitution has been known (Japanese Unexamined Patent Application, First Publication No. 2010-172295, Japanese Unexamined Patent Application, First Publication No. 2007-143409, Japanese Unexamined Patent Application, First Publication No. 2007-043910, Japanese Unexamined Patent Application, First Publication No. 2008-253182, Japanese Unexamined Patent Application, First Publication No. 2019-088326, Japanese Unexamined Patent Application, First Publication No. 2019-088327, PCT International Publication No. WO2005/116206, PCT International Publication No. WO2012/164933, PCT International Publication No. WO2012/169203, PCT International Publication No. WO2015/186298, and the like).

In the method of the present embodiment, a microorganism into which these improved-type nitrile hydratases have been introduced can also be used.

These microorganisms having nitrile hydratase activity or the treated product thereof can be used in an amide synthesis reaction immediately after preparation of the bacterial cell, and can also be stored after the preparation of the bacterial cell and used in the amide synthesis reaction as necessary. A culture method of the microorganism for preparing the bacterial cell can be appropriately selected depending on the kind of the microorganism. A seed culture may be carried out before the main culture.

The microbial cell body having nitrile hydratase activity or the treated product thereof can also be used in a batch reaction or a continuous reaction. In addition, an appropriate reaction form such as a fluidized bed, a fixed bed, or a suspension bed can be selected. At this time, a biocatalyst temperature in the reaction solution is not particularly limited as long as it does not hinder the mixing of the aqueous medium and the nitrile compound.

### (2) Nitrile compound

The nitrile compound used as a raw material in the production method according to the present embodiment is not particularly limited as long as it is a compound which is converted into an amide compound by the biocatalyst having nitrile hydratase activity. Examples thereof include an aliphatic saturated nitrile such as acetonitrile, propionitrile, succinonitrile, and adiponitrile; an aliphatic unsaturated nitrile such as acrylonitrile and methacrylonitrile; an aromatic nitrile such as benzonitrile and phthalonitrile; and a heterocyclic nitrile such as nicotinonitrile. The nitrile compound in the present embodiment is preferably a nitrile compound having 2 to 4 carbon atoms, such as acetonitrile, propionitrile, acrylonitrile, methacrylonitrile, n-butyronitrile, and isobutyronitrile; and particularly acrylonitrile, methacrylonitrile, or acetonitrile from the viewpoint of exhibiting the effect in the present embodiment.

### (3) Raw water

Water used as a raw material (raw water) is used in the hydration reaction with acrylonitrile when producing acrylamide. Examples of the water include pure water and an aqueous solution in which an acid, a salt, or the like is dissolved in water. Examples of the acid include phosphoric acid, acetic acid, citric acid, boric acid, acrylic acid, and formic acid. Examples of the salt include a sodium salt, a potassium salt, and an ammonium salt of the above-described acid. Specific examples of the water are not particularly limited, but include water such as pure water, ultrapure water, and tap water; and buffer solutions such as a Tris buffer solution, a phosphate buffer solution, an acetate buffer solution, a citrate buffer solution, and a borate buffer solution. The pH of the raw water at 20°C is preferably 5 to 9.

### (4) Production of amide compound from nitrile compound using biocatalyst

In the method for producing an amide compound from the nitrile compound using the biocatalyst having nitrile hydratase activity according to the present embodiment, any of the following reactions (i) to (iii) can also be adopted.
(i) A method in which all of reaction raw materials including the biocatalyst, acrylonitrile, and raw water are charged into a reactor at once, and a reaction is performed (batchwise reaction)
(ii) A method in which part of the reaction raw materials is charged into a reactor, and the remaining reaction raw materials are continuously or intermittently supplied thereto to perform a reaction (semi-batch reaction)
(iii) A method in which a reaction mixture is continuously produced without extracting the entire amount of reaction mixture in a reactor, while continuously or intermittently supplying the reaction raw materials and continuously or intermittently taking out the reaction mixture containing the reaction raw materials and produced acrylamide (continuous reaction)

The type of reactor is not particularly limited, and for example, various types of reactors such as a stirring type, a fixed bed type, a fluidized bed type, a moving bed type, a column type, and a tubular type can be used. Among the above, a stirring type in which dispersion and mixing of the raw material can be promoted is preferable. Reactors having different forms can also be connected in combination.

The apparatus used in the multi-stage continuous reaction includes two or more reactors connected in series, and produces, in each reactor, the amide compound from the nitrile compound and water by a continuous reaction using the biocatalyst. More specifically, in the continuous reaction apparatus, the raw material is added to a reactor located at the most upstream position and connected to the reactor for the first time to initiate a reaction, and the reaction proceeds while moving the reaction solution to a reactor located sequentially on the downstream side. Next, the reaction solution containing the amide compound produced from the reactor located at the most downstream, that is, the target amide compound aqueous solution may be collected. The biocatalyst may be separated from the recovered reaction solution and supplied to the reactor again.

The number of reactors (reaction vessels) is not particularly limited, and can be appropriately selected depending on the reaction conditions and the like. For example, the number of reactors is preferably 2 to 20, more preferably 2 to 12, and still more preferably 2 to 10. The reactors may be present in parallel connection as necessary. The reactors may be independent of each other, or may be a large reactor partitioned into a plurality of reactors by a partition wall. With the reactors partitioned by a partition wall, each space partitioned by the partition wall is regarded as one reactor.

A tank for supplying the nitrile compound, the biocatalyst, the raw water, and other auxiliary agents is not limited to one tank at the topmost stream, and may be one tank or a plurality of two or more tanks. The latter (downstream) tank is used for reaction push cutting and aging, and a reaction solution containing a product can be extracted from the tank at the lowermost stream (final tank) or the tank present upstream of the lowermost stream.

The number of tanks for supplying the raw material and the number of tanks for aging and the like can be appropriately selected depending on the reaction conditions, the reaction scale, and the like.

A stirring blade is preferable as a stirrer. The shape of the stirring blade is also not particularly limited, and examples thereof include a paddle, a disc turbine, a propeller, a helical ribbon, an anchor, and a fouler.

A water-soluble monocarboxylate having 2 or more carbon atoms can be added to the reaction solution. The timing of adding the water-soluble monocarboxylate is not particularly limited, and the water-soluble monocarboxylate can be added to the reactor located on the most upstream side so that the water-soluble monocarboxylate contained in the reaction solution moves to the downstream side together with the reaction solution, thereby being contained in the reaction solution in each reactor. In addition, the water-soluble monocarboxylate may be added to each reactor before the reaction is started or after the reaction is started.

By adding the water-soluble monocarboxylate having 2 or more carbon atoms, stability of the acrylamide in the reaction solution can be improved.

The water-soluble monocarboxylate may be a saturated monocarboxylate or an unsaturated monocarboxylate. Examples of the saturated carboxylic acid include acetic acid, propionic acid, and n-caproic acid. Examples of the unsaturated carboxylic acid include acrylic acid and methacrylic acid. Examples of the salt include a sodium salt, a potassium salt, and an ammonium salt of the saturated monocarboxylic acid or the unsaturated monocarboxylic acid. These water-soluble monocarboxylates can be used alone or in combination of two or more kinds thereof.

The addition amount of water-soluble monocarboxylate is preferably 20 to 5,000 mg/kg with respect to the acrylamide to be produced.

### <Control of pH of reaction solution>

In the present embodiment, regarding the pH of the reaction solution in which acrylonitrile is hydrated to produce acrylamide, the pH of the reaction solution is increased in the middle of the hydration reaction. By increasing the pH of the reaction solution in the middle of the hydration reaction, foaming of the obtained amide compound aqueous solution can be suppressed and foamability can be lowered, and thus handling of the reaction solution is facilitated.

As a method of measuring the pH of the reaction solution, a known method can be adopted; and examples thereof include an indicator method, a metal electrode method, a glass electrode method, and a semiconductor sensor method. In the present embodiment, measurement by a glass electrode method widely used in the industry is preferable.

In one example of the embodiment, for example, in the middle of the hydration reaction, the pH of the reaction solution is increased to be higher than the pH at the start of the reaction.

The pH of the reaction solution at the start of the reaction can be set to, for example, 6.6 to 7.5, preferably 6.8 to 7.5, more preferably 6.9 to 7.4, and still more preferably 7.0 to 7.3. By setting the pH of the reaction solution to be within the above-described range at the start of the reaction, the amide compound can be efficiently obtained from the nitrile compound.

The range of increase of the pH when increasing the pH of the reaction solution is preferably 0.3 to 1.5, more preferably 0.4 to 1.4, and still more preferably 0.5 to 1.2. When the range of increase of the pH is within the above-described range, it is easy to suppress foaming of the obtained amide compound aqueous solution, and there tends to be a high effect of improving the efficiency of the hydration reaction.

In a suitable example, the pH in at least part of the latter half of the reaction is set to be higher than the pH of the reaction solution in the former half of the reaction to perform the hydration reaction.

Here, the "former half of the reaction" refers to "from the start time of the reaction to a time at which the concentration of the amide compound in the reaction solution is less than 40% by mass"; and the "latter half of the reaction" refers to "from the time at which the concentration of the amide compound in the reaction solution is 40% by mass or more or more than 40% by mass". In other words, a period in which the contained amount of amide compound in the product with respect to the total mass of the reaction solution is less than 40% by mass is referred to as the former half of the reaction; and a period in which the contained amount of amide compound in the product with respect to the total mass of the reaction solution is 40% by mass or more or more than 40% by mass is referred to as the latter half of the reaction. The total mass of the reaction solution refers to the mass of the entire reaction solution in the reactor of interest, and includes the mass of the amide compound or the nitrile compound.

By setting the pH of the reaction solution in at least part of the latter half of the reaction to be higher than the pH of the reaction solution in the former half of the reaction, the reaction can be performed without lowering the reaction efficiency, and foaming of the obtained amide compound aqueous solution can be suppressed and the foamability can be lowered, and thus handling of the reaction solution is facilitated.

The pH of the reaction solution in the latter half of the reaction may be higher than the pH of the reaction solution in the former half of the reaction only in part of the latter half of the reaction, or may be higher than the pH of the reaction solution in the former half of the reaction over the entire latter half of the reaction. Among these, since it is easy to lower the foamability of the obtained amide compound aqueous solution, it is preferable that the pH of the reaction solution in the latter half of the reaction be set to be higher than the pH of the reaction solution in the former half of the reaction over the entire latter half of the reaction.

The pH of the reaction solution in the former half of the reaction can be set to, for example, 6.6 to 7.5, preferably 6.8 to 7.5, more preferably 6.9 to 7.4, and still more preferably 7.0 to 7.3. By setting the pH of the reaction solution in the former half of the reaction to 6.6 to 7.5, the amide compound can be efficiently obtained from the nitrile compound.

The pH of the reaction solution in the latter half of the reaction can be set to, for example, 7.5 or more and less than 8.5, preferably 7.5 to 8.4, more preferably 7.6 to 8.3, and still more preferably 7.8 to 8.3. By setting the pH of the reaction solution in the latter half of the reaction to 7.5 or more, foaming of the reaction solution can be sufficiently suppressed. By setting the pH of the reaction solution in the latter half of the reaction to less than 8.5, it is possible to efficiently produce the amide compound.

In the present embodiment, when the pH of the reaction solution in the latter half of the reaction is higher than the pH of the reaction solution in the former half of the reaction, the difference therebetween is not limited, but, for example, the difference between the pH of the reaction solution in the latter half of the reaction and the pH of the reaction solution in the former half of the reaction can be set to 0.3 to 1.5, preferably 0.4 to 1.4 and more preferably 0.5 to 1.2.

In the present embodiment, a method of adjusting the pH of the reaction solution is not limited. The pH of the reaction solution can be adjusted by appropriately adding an acid or a base to the reaction solution, depending on the pH of the reaction solution.

As the acid, an inorganic acid or an organic acid can be used. Examples of the inorganic acid include halogenated acids such as hydrochloric acid, hydrobromic acid, and hydriodic acid; halogenated oxoacids such as hypochlorous acid, chloric acid, hypobromous acid, and hypoiodic acid; sulfuric acid, nitric acid, phosphoric acid, and boric acid. Examples of the organic acid include carboxylic acids such as formic acid, acetic acid, propionic acid, acrylic acid, methacrylic acid, crotonic acid, oxalic acid, malonic acid, fumaric acid, maleic acid, citric acid, lactic acid, and benzoic acid; and sulfonic acids such as methanesulfonic acid, ethanesulfonic acid, benzenesulfonic acid, and p-toluenesulfonic acid.

When these acids are used, the acid can be used in any of a gaseous state, a solid state, or a liquid state; but in consideration of the ease of supply to the reactor, it is preferable to use the acid in a liquid or solid state. The concentration of the acid when used as a liquid is not particularly limited, and can be appropriately selected.

As the base, an inorganic base or an organic base can be used. Examples of the inorganic base include hydroxides of an alkali metal, such as lithium hydroxide, sodium hydroxide, and potassium hydroxide; hydroxides of an alkaline earth metal, such as magnesium hydroxide and calcium hydroxide; carbonates of an alkali metal, such as lithium carbonate, sodium carbonate, and potassium carbonate; hydrogen carbonates of an alkali metal, such as lithium hydrogen carbonate, sodium hydrogen carbonate, and potassium hydrogen carbonate; and ammonia. Examples of the organic base include trimethylamine, triethylamine, aniline, and pyridine.

When these bases are used, the base can be used in any of a gaseous state, a solid state, or a liquid state; but in consideration of the ease of supply to the reactor, it is preferable to use the base in a liquid or solid state. The concentration of the base when used as a liquid is not particularly limited, and can be appropriately selected.

A reaction temperature when hydrating the acrylonitrile, that is, a temperature of the reaction solution is not particularly limited; but is preferably 10°C to 50°C, more preferably 15°C to 40°C, and still more preferably 20°C to 35°C. By setting the reaction temperature to be 10°C or higher, the reaction activity of the biocatalyst can be sufficiently increased. In addition, by setting the reaction temperature to be 50°C or lower, inactivation of the biocatalyst can be prevented. In addition, in order to reduce a heat removal load of the reactor, it is preferable that the water or acrylonitrile be supplied at a temperature lower than the reaction temperature by 5°C or higher.

The amount of catalyst is actually compared in terms of unit, not in terms of the actual weight of the catalyst. In terms of nitrile hydratase activity, the amount of enzyme for purifying 1 µmol of acrylamide in 1 minute is defined as 1 U. Examples of the specific activity of the nitrile hydratase in the present embodiment include 50 U/mg or more, preferably 80 U/mg or more, and more preferably 100 U/mg or more on a dry cell basis.

### Examples

Hereinafter, the present invention will be described in more detail with reference to Examples and Comparative Example, but the present invention is not limited only to Examples described below. In Examples, "%" indicates "% by mass".

### [Preparation of biocatalyst]

### <Preparation of bacterial cell catalyst derived from Rhodococcus rhodochrous J1 strain>

### Pre-culture conditions:

### (Culture medium formulation)

Fructose: 2%, Polypepton: 5% (Nippon Pharmaceutical Co., Ltd.), Yeast extract: 0.3% (Oriental Yeast Co., ltd.), KH₂PO₄: 0.1%, K₂HPO₄: 0.1%, MgSO₄·H₂O: 0.1%, pH: 7

### (Culture method)

100 mL of the culture medium was dispensed into a 500 mL Erlenmeyer flask, plugged with a cotton plug, and sterilized in an autoclave at 121°C for 20 minutes. Rhodococcus rhodochrous J1 (FERM BP-1478) was inoculated, shaken, and cultured at 30°C for 48 hours.

### Main culture conditions:

### (Culture medium formulation)

Initial medium; Yeast extract: 0.2%, KH₂PO₄: 0.1%, K₂HPO₄: 0.1%, MgSO₄·7H₂O: 0.1%, CoCl₂·6H₂O: 0.002%, sulfuric acid: 0.025%, fructose: 2%, urea: 2%, ethanol: 0.4%, Pluronic L61: 0.1% (Asahi Denka Kogyo Co., Ltd.), pH: 7 "Pluronic" is a registered trademark.

Post-addition culture medium; fructose: 20%, ethanol: 5%, sulfuric acid: 6%, pH: 6.5

### (Culture method)

2 L of the initial medium was dispensed into a 3 L mini jar fermenter, and sterilized in an autoclave at 121°C for 20 minutes. Here, fructose, ethanol, and urea were separately filtered aseptically and added to the culture medium. For the filtration, a filter paper having a pore size of 0.45 µm manufactured by Advantech Toyo Co., Ltd. was used.

After culturing at an in-vessel pressure of 0.098 MPa, a stirring speed of 600 rpm, an aeration amount of 1 vvm, a pH of 7, and a temperature of 30°C for 43 hours, the culture medium was washed with a 50 mM phosphate buffer (pH: 7.7) to obtain a bacterial cell suspension having a dry cell weight of 15%.

### <Example 1>

Seven reactors (internal volume: 1.3 L) equipped with jacket coolers were connected in series so that a reaction solution flowed from the first tank to the seventh tank in order.

In the first tank, a 50 mM phosphate buffer solution (pH: 7.0) was continuously supplied at 525 mL/hr, acrylonitrile was continuously supplied 150 mL/hr, and a diluted bacterial cell suspension obtained by diluting the bacterial cell suspension having a dry cell weight of 15% 40-fold with a 50 mM phosphate buffer solution was continuously supplied at 100 mL/hr. Only acrylonitrile was continuously supplied to the second tank at 120 mL/hr; only acrylonitrile was continuously supplied to the third tank at 120 mL/hr; and only acrylonitrile was continuously supplied to the fourth tank at 80 mL/hr, thereby initiating the reaction.

A height level of the overflow pipe through which the reaction solution in each tank flowed out was adjusted so that the amount of reaction solution in each of the first to seventh tanks was 1 L, and the reaction solution was fed to the next tank by overflow. The temperature was controlled using cooling water (10°C) of the jacket such that the reaction solution temperatures of the first to seventh tanks were 20°C.

Using a two-paddle wing (wing diameter: 350 mm, wing width: 100 mm), the stirring power per fluid of the reaction solution in all reactors from the first tank to the seventh tank was adjusted to 0.08 kW/m³ (fluid number: 0.057). Here, the required stirring power per fluid of the reaction solution was calculated by dividing the required stirring power in each reactor by the liquid amount (1 L = 0.001 m³).

The pH of the reaction solution in each tank was measured with a KCl supplytype pH detector, and a 0.06 N sodium hydroxide aqueous solution was automatically added thereto so that the pH was set to a set value.

The pH of the reaction solution in each of the first, second, third, fourth, fifth, sixth, and seventh tanks was set to 7.2, 7.2, 7.2, 7.5, 7.7, 8.0, and 8.0, respectively.

One day after the start of the reaction, after confirming that the pH of each reactor was maintained at the set value, the acrylamide concentration of the reaction solution in each reactor was measured with a refractometer (manufactured by Atago Co., Ltd.; RX-5000). The acrylamide concentrations in the first, second, third, fourth, fifth, sixth, and seventh tanks were 19%, 31%, 38%, 43%, 49%, 50%, and 50%, respectively; and the acrylamide concentration of the reaction solution flowing out from the seventh tank reached the target concentration of 50%. In addition, the acrylonitrile concentration of the reaction solution in the seventh tank was measured by gas chromatography (column: 1 m of PoraPak-PS manufactured by Waters Corporation; 180°C; carrier gas: helium; detector: FID). The unreacted acrylonitrile (AN) in the seventh tank was 10 ppm, and the reaction efficiency was high. It is preferable that the unreacted acrylonitrile concentration be 100 ppm or less from the viewpoint of further improving the quality of the polymerization of acrylamide.

Next, 300 mL of the reaction solution in the seventh tank was placed in a 500 mL graduated cylinder, and allowed to stand in a constant-temperature tank at 25°C for 10 minutes. A glass ball filter (Kinoshita glass filter 504G) was placed at a position of 5 mm from the bottom of the center portion of the graduated cylinder, and air with a pressure of 0.5 kg/cm³ was passed through at 800 cc/min. When the reaction solution began to foam, the aeration was stopped at a point where the height of the bubbles was stable, and the time until the bubbles disappeared was measured. As the time until the bubbles disappeared became shorter, the amount of protein brought from the biocatalyst into the acrylamide aqueous solution became smaller. It is required for the time until the bubbles disappeared to be within 30 seconds in terms of quality. The time until the bubbles disappeared was 5 seconds, which satisfied the required quality.

### <Example 2>

The reaction was carried out in the same manner as in Example 1, except that the pH of the reaction solution in the first, second, third, fourth, fifth, sixth, and seventh tanks was set to 6.8, 7.0, 7.5, 8.3, 8.3, 8.3, and 8.3, respectively.

One day after the start of the reaction, after confirming that the pH of each reactor was maintained at the set value, the acrylamide concentration of the reaction solution in each reactor was measured; and as a result, the acrylamide concentrations of the first, second, third, fourth, fifth, sixth, and seventh tanks were 18%, 31%, 36%, 42%, 48%, 50%, and 50%, respectively, and the acrylamide concentration of the reaction solution flowing out from the seventh tank reached the target concentration of 50%. In addition, as a result of measuring the acrylonitrile concentration of the reaction solution in the seventh tank, the unreacted acrylonitrile concentration was 20 ppm, and the reaction efficiency was high.

As a result of measuring the defoaming time using the reaction solution in the seventh tank, the defoaming time was 2 seconds, which satisfied the required quality.

### <Example 3>

The reaction was carried out in the same manner as in Example 1, except that the pH of the reaction solution in the first, second, third, fourth, fifth, sixth, and seventh tanks was set to 6.8, 6.8, 6.8, 8.3, 8.3, 8.3, and 8.3, respectively.

One day after the start of the reaction, after confirming that the pH of each reactor was maintained at the set value, the acrylamide concentration of the reaction solution in each reactor was measured; and as a result, the acrylamide concentrations of the first, second, third, fourth, fifth, sixth, and seventh tanks were 18%, 30%, 35%, 41%, 47%, 49%, and 50%, respectively, and the acrylamide concentration of the reaction solution flowing out from the seventh tank reached the target concentration of 50%. In addition, as a result of measuring the acrylonitrile concentration of the reaction solution in the seventh tank, the unreacted acrylonitrile concentration was 30 ppm, and the reaction efficiency was high.

As a result of measuring the defoaming time using the reaction solution in the seventh tank, the defoaming time was 2 seconds, which satisfied the required quality.

### <Example 4>

The reaction was carried out in the same manner as in Example 1, except that the pH of the reaction solution in the first, second, third, fourth, fifth, sixth, and seventh tanks was set to 7.5, 7.5, 7.5, 8.5, 8.5, 8.5, and 8.5, respectively.

One day after the start of the reaction, after confirming that the pH of each reactor was maintained at the set value, the acrylamide concentration of the reaction solution in each reactor was measured; and as a result, the acrylamide concentrations of the first, second, third, fourth, fifth, sixth, and seventh tanks were 18%, 30%, 34%, 41%, 47%, 49%, and 50%, respectively, and the acrylamide concentration of the reaction solution flowing out from the seventh tank reached the target concentration of 50%. In addition, as a result of measuring the acrylonitrile concentration of the reaction solution in the seventh tank, the unreacted acrylonitrile concentration was 300 ppm, and the reaction efficiency was higher in Examples 1 to 3 in which the pH after the latter half of the reaction was controlled to be less than 8.5.

As a result of measuring the defoaming time using the reaction solution in the seventh tank, the defoaming time was 2 seconds, which satisfied the required quality.

### <Comparative Example 1>

The reaction was carried out in the same manner as in Example 1, except that the pH of the reaction solution in the first, second, third, fourth, fifth, sixth, and seventh tanks was set to 7.0.

One day after the start of the reaction, after confirming that the pH of each reactor was maintained at the set value, the acrylamide concentration of the reaction solution in each reactor was measured; and as a result, the acrylamide concentrations of the first, second, third, fourth, fifth, sixth, and seventh tanks were 19%, 31%, 36%, 44%, 49%, and 50%, respectively, and the acrylamide concentration of the reaction solution flowing out from the seventh tank reached the target concentration of 50%. In addition, as a result of measuring the acrylonitrile concentration of the reaction solution in the seventh tank, the unreacted acrylonitrile concentration was not detected, which satisfied the required quality.

As a result of measuring the defoaming time using the reaction solution in the seventh tank, the defoaming time was 150 seconds, which did not satisfy the required quality.

### <Comparative Example 2>

The reaction was carried out in the same manner as in Example 1, except that the pH of the reaction solution in the first, second, third, fourth, fifth, sixth, and seventh tanks was set to 7.5.

One day after the start of the reaction, after confirming that the pH of each reactor was maintained at the set value, the acrylamide concentration of the reaction solution in each reactor was measured; and as a result, the acrylamide concentrations of the first, second, third, fourth, fifth, sixth, and seventh tanks were 18%, 30%, 36%, 43%, 49%, 50%, and 50%, respectively, and the acrylamide concentration of the reaction solution flowing out from the seventh tank reached the target concentration of 50%. In addition, as a result of measuring the acrylonitrile concentration of the reaction solution in the seventh tank, the unreacted acrylonitrile concentration was 10 ppm, which satisfied the required quality.

As a result of measuring the defoaming time using the reaction solution in the seventh tank, the defoaming time was 80 seconds, which did not satisfy the required quality.

### <Comparative Example 3>

The reaction was carried out in the same manner as in Example 1, except that the pH of the reaction solution in the first, second, third, fourth, fifth, sixth, and seventh tanks was set to 8.0.

One day after the start of the reaction, after confirming that the pH of each reactor was maintained at the set value, the acrylamide concentration of the reaction solution in each reactor was measured; and as a result, the acrylamide concentrations of the first, second, third, fourth, fifth, sixth, and seventh tanks were 17%, 30%, 35%, 41%, 48%, 50%, and 50%, respectively, and the acrylamide concentration of the reaction solution flowing out from the seventh tank reached the target concentration of 50%. In addition, as a result of measuring the acrylonitrile concentration of the reaction solution in the seventh tank, the unreacted acrylonitrile concentration was 120 ppm, which did not slightly satisfy the required quality.

As a result of measuring the defoaming time using the reaction solution in the seventh tank, the defoaming time was 45 seconds, which did not satisfy the required quality.

**[Table 1]**

| Reactor | | 1 | 2 | 3 | 4 | 5 | 6 | 7 | Reactivity | Foam breakability |
|---|---|---|---|---|---|---|---|---|---|---|
| [Example 1] | AAM [%] | 19 | 31 | 38 | 43 | 49 | 50 | 50 | O AN 10 ppm | O 5 seconds |
| | pH | 7.2 | 7.2 | 7.2 | 7.5 | 7.7 | 8.0 | 8.0 | | |
| [Example 2] | AAM [%] | 18 | 31 | 36 | 42 | 48 | 50 | 50 | O AN 20 ppm | O 2 seconds |
| | pH | 6.8 | 7.0 | 7.5 | 8.3 | 8.3 | 8.3 | 8.3 | | |
| [Example 3] | AAM [%] | 18 | 30 | 35 | 41 | 47 | 49 | 50 | O AN 30 ppm | O 2 seconds |
| | pH | 6.8 | 6.8 | 6.8 | 8.3 | 8.3 | 8.3 | 8.3 | | |
| [Example 4] | AAM [%] | 18 | 30 | 34 | 41 | 47 | 49 | 50 | X AN 300 ppm | O 2 seconds |
| | pH | 7.5 | 7.5 | 7.5 | 8.5 | 8.5 | 8.5 | 8.5 | | |
| [Comparative Example 1] | AAM [%] | 19 | 31 | 36 | 44 | 49 | 50 | 50 | O not detected | X 150 seconds |
| | pH | 7.0 | 7.0 | 7.0 | 7.0 | 7.0 | 7.0 | 7.0 | | |
| [Comparative Example 2] | AAM [%] | 18 | 30 | 36 | 43 | 49 | 50.0 | 50.0 | O AN 10 ppm | X 80 seconds |
| | pH | 7.5 | 7.5 | 7.5 | 7.5 | 7.5 | 7.5 | 7.5 | | |
| [Comparative Example 3] | AAM [%] | 17 | 30 | 35 | 41 | 48 | 50 | 50 | X AN 120 ppm | X 45 seconds |
| | pH | 8.0 | 8.0 | 8.0 | 8.0 | 8.0 | 8.0 | 8.0 | | |

The results of Examples 1 to 3 are summarized as follows. In the former half of the reaction (the first tank, the second tank, and the third tank) in which the contained amount of amide compound with respect to the total mass of the reaction solution in each reactor was less than 40% by mass, the pH of the reaction solution was set to the first pH to perform the hydration reaction of the nitrile compound. Due to the hydration reaction, the concentration of the amide compound in the reaction solution increased as the reaction solution progressed from the first tank to the third tank. The contained amount of amide compound with respect to the total mass of the reaction solution was 40% by mass or more from the third tank to the fourth tank, and in the latter half of the reaction after the fourth tank, the pH of the reaction solution was set to the second pH, and the hydration reaction was continued. Here, a condition in which the second pH was higher than the first pH was satisfied. As a result, in any of Examples 1 to 4, the aqueous solution of acrylamide obtained in the seventh tank had suppressed foaming and low foamability. In addition, in Examples 1 to 3 in which the second pH in the latter half of the reaction was controlled to be less than 8.5, the unreacted acrylonitrile concentration was lower and the reaction efficiency was higher than in Example 4 in which the second pH was 8.5.

### INDUSTRIAL APPLICABILITY

The present invention is useful in the industrial production of an amide compound such as acrylamide and methacrylamide.

## Claims

1. A method for producing an amide compound, comprising:
performing a hydration reaction on a nitrile compound in the presence of a biocatalyst having nitrile hydratase activity,
wherein the pH of a reaction solution is increased in a middle of the hydration reaction.

2. The method for producing an amide compound according to Claim 1,
wherein the pH of the reaction solution is increased in the middle of the reaction to be higher than the pH at the start of the reaction.

3. The method for producing an amide compound according to Claim 1 or 2,
wherein the hydration reaction is performed in a state in which the pH of the reaction solution in at least part of the latter half of the reaction, at which a contained amount of amide compound is 40% by mass or more with respect to a total mass of the reaction solution, is set to be higher than the pH of the reaction solution in the former half of the reaction, at which the contained amount of amide compound is less than 40% by mass with respect to the total mass of the reaction solution.

4. The method for producing an amide compound according to any one of Claims 1 to 3,
wherein the pH of the reaction solution is increased by 0.3 to 1.5.

5. The method for producing an amide compound according to any one of Claims 1 to 4,
wherein the pH of the reaction solution at a start of the reaction is 6.6 to 7.5.

6. The method for producing an amide compound according to any one of Claims 3 to 5,
wherein the hydration reaction is performed in a state in which the pH of the reaction solution in the former half of the reaction is set to 6.6 to 7.5 and the pH of the reaction solution in the latter half is set to 7.5 or more and less than 8.5.

7. The method for producing an amide compound according to Claim 1 or 2, wherein the nitrile compound is acrylonitrile or methacrylonitrile.

8. The method for producing an amide compound according to Claim 1 or 2, wherein the amide compound is acrylamide or methacrylamide.
